# EUROPEAN PATENT SPECIFICATION

(11) **EP 2 296 617 B1**
(45) Date of publication and mention of the grant of the patent: **07.08.2013**
(21) Application number: 09772287.0
(22) Date of filing: 10.06.2009
(51) Int. Cl.: A61K 8/67, A61Q 17/04, A61Q 19/00, A61K 8/88

(54) **NIACINAMIDE CONTAINING COSMETIC COMPOSITIONS WITH IMPROVED SKINFEEL PROPERTIES**
NIACINAMID ENTHALTENDE KOSMETISCHE ZUSAMMENSETZUNGEN MIT VERBESSERTEM HAUTGEFÜHL
COMPOSITIONS COSMÉTIQUES CONTENANT DE LA NIACINAMIDE, DOTÉES DE PROPRIÉTÉS DE TOUCHER DE PEAU AMÉLIORÉES

(30) Priority: 30.06.2008 US 164138
(43) Date of publication of application: 23.03.2011
(73) Proprietor: Unilever PLC, London Greater London EC4P 4BQ (GB); Unilever N.V., 3013 AL Rotterdam (NL)
(72) Inventor: POLONKA, Jack, Trumbull, Connecticut 06611 (US); WEI, Xiaoling, Trumbull, Connecticut 06611 (US); BARTOLONE, John, Brian, Trumbull, Connecticut 06611 (US)
(74) Representative: Acham, Nicholas Clive
(86) International application number: PCT/EP2009/057156
(87) International publication number: WO 2010/000587

(56) References cited:
- WO-A1-2008/006739
- FR-A1- 2 910 279
- US-A1- 2007 009 474
- US-B1- 6 551 604

## Description

### BACKGROUND OF THE INVENTION

### Field of the Invention

The invention concerns improvements in skinfeel of niacinamide containing cosmetic compositions that incorporate carboxylic functionalized structurants as thickeners.

### The Related Art

Invariably cosmetic compositions are formulated with one or more structurants to thicken the system. A significant number of formulas utilize structurants having carboxylic acid (or salt thereof) functional groups. Illustrative structurants are C₁₂-C₂₀ fatty acids, acrylic acid functionalized polymers, and salt derivatives resulting from neutralization of the carboxylic acid groups.

Niacinamide is a substance found in many cosmetics as a skin lightening and/or moisturizing agent. Problems can arise from interaction of niacinamide with the carboxylic acid or salt bearing structurants. For instance, niacinamide and polyacrylic acids/salts may adversely interact resulting in phase separation between these materials. In many instances, crystallization may occur. Niacinamide and the carboxylic acid/salt structurant can form mixed crystals or a complex together. This decreases water dispersability of the carboxylic containing polymer or of the long chain fatty acid. Of particular concern are vanishing creams. These are formulated with high levels of stearic acid suspended in water by an emulsifying agent such as an alkali metal soap. Niacinamide can turn the vanishing cream into a hardened aesthetically unacceptable substance.

Cosmetic compositions with niacinamide have been reported by Procter & Gamble in a series of patent documents which include: US 5 833 998; US 5 939 082; US 5 962 482; US 5 968 528; US 5 980 921; US 5 997 890; US 5 997 887; US 6 024 942; US 6183761 B1; US 6 224 888; US 6 309 657 B2; and US 6 447 647 B1. Vanishing creams have been particularly investigated by Unilever. Related documents include US 3 938 810; US 4 096 240; US 6 939 552 B2; GB 2 230186; and EP 0 396 422.

The present invention is focused upon cosmetic compositions with niacinamide and carboxylic acid/salt structurants that overcome problems of poor skinfeel arising from composition hardening and phase separation. It is also a focus of the present invention to improve levels of photoprotection for cosmetic compositions of the aforementioned type which contain sunscreens.

### SUMMARY OF THE INVENTION

A cosmetic composition is provided which includes:
(i) from 0.5 to 10% by weight of niacinamide;
(ii) from 0.1 to 30% by weight of a structurant selected from the group consisting of C₁₂-C₂₀ fatty acid or salt thereof, a polymer with repeating carboxylic acid or salt thereof monomer units, and mixtures thereof; and
(iii) from 0.1 to 20% by weight of particles comprising a condensation polymerized polyamide, the polyamide having an amine value of no lower than 0.3 and an HLB value of 16 or higher, wherein a sunscreen agent is either solubilized or uniformly dispersed within the condensation polymerized polyamide to form composite particles.

### DETAILED DESCRIPTION OF THE INVENTION

Now it has been found that particles of certain condensation polymerized polyamides can inhibit the adverse interaction of niacinamide with higher molecular weight carboxylic acid or salt functionalized polymers or of fatty acids. Good skinfeel aesthetics can be achieved. The polyamide must be a material having an amine value no lower than 0.3 and an HLB value of 16 or higher.

### NIACINAMIDE

Niacinamide is also known as vitamin B3. Amounts of niacinamide within cosmetic compositions of the present invention will range from 0.5 to 10%, preferably from 1 to 8%, and optimally from 3 to 7% by weight of the composition.

### CARBOXYLIC STRUCTURANT

Compositions of the present invention will have a carboxylic stucturant to function as a thickener. The structurant may be selected from C₁₂-C₂₀ fatty acid or salts thereof, polymers with monomeric units having at least one carboxylic acid or salt thereof and combinations thereof. By the term "salt" is meant an alkali metal, ammonium or organoammonium cation. Illustrative cations are sodium, potassium, lithium, ammonium, trimethylammonium and triethanolammonium cations.

When the C₁₂-C₂₀ fatty acid is utilized as a structurant, it may be selected from but not limited to stearic acid, oleic acid, isostearic acid, linoleic acid, palmitic acid, cetearic acid, myristic acid and behenic acid. Suitable C₁₂-C₂₀ fatty acid salts include sodium stearate, potassium stearate, ammonium stearate, triethanolammonium stearate, sodium behenate, sodium palmitate, potassium oleate and potassium myristate. Stearic acid is particularly preferred. Amounts of fatty acid may range from 5 to 30%, preferably from 7 to 28%, more preferably from 10 to 25%, optimally from 12 to 20% by weight of the composition.

Vanishing cream cosmetic compositions are particularly relevant to the present invention. These vanishing creams will have relatively high levels of fatty acid (e.g. above 7% and ordinarily 12-20%). Generally, vanishing creams will include both a C₁₂-C₂₀ fatty acid and also in smaller amounts an alkali metal salt of the fatty acid such at levels from 0.1 to 20% but preferably from 0.5 to 4% by weight of the composition.

Structurants according to the present invention may be in polymeric form. These polymers will contain at least one carboxylic acid or salt thereof functionality in one or more of the monomeric units constituting the polymer. The polymer may be a homo or co-polymer. Weight average molecular weight may range from 800 to 5 million, preferably from 2,000 to 1 million, optimally from 5,000 to 100,000. Typically the monomeric units may be selected from acrylic acid, methacrylic acid, maleic acid, crotonic acid, adipic acid as well as the acid salts thereof, and combinations thereof. Illustrative but not limiting are polyacrylic acids/salts, polymethacyrlic acids/salts, and polyvinylmethylether/maleic acid, all of which may be crosslinked or non-crosslinked.

Examples of commercially available carboxylic acid polymers useful herein include the Carbomers, which are homopolymers of acrylic acid crosslinked with allyl ethers of sucrose or pentaerytritol. The Carbomers are available as the Carbopol® 900 series from Noveon Corporation (e.g. Carbopol® 954). In addition, other suitable carboxylic acid polymeric agents include copolymers of C₁₀₋₃₀ alkyl acrylates with one or more monomers of acrylic acid, methacrylic acid, or one of their short chain (i.e. C₁₋₄ akohol) esters, wherein the crosslinking agent is an allyl ether of sucrose or pentaerythritol. These copolymers are known as acrylates/C₁₀₋₃₀ alkyl acrylate crosspolymers and are commercially available as Carbopol® 1342, Carbopol® 1382, Ultrez® 21, Pemulen® TR-1, and Pemulen® TR-2 from Noveon division of Lubrizol Corporation.

Additionally, useful co-polymers are illustrated but not limited by the following examples: hydroxyethyl acrylate/sodium acryloyldimethyl taurate (e.g. Simulgel® NS and INS 100) and acrylate/sodium acryloyldimethyl taurate (e.g. Simulgel® EG). Amounts of the polymeric carboxylic acid/salt containing structurants may range from 0.1 to 10%, preferably from 0.3 to 5%, and even more preferably from 0.25 to 4% by weight of the composition.

### POLYAMIDE PARTICLES

An important component of compositions of the present invention is that of particles of a condensation polymerized polyamide (sometimes referred to as a resin herein). A preferred embodiment of the resin is a polyalkyleneoxypolyamide (referred to as an PAOPA resin) and also an ester-terminated poly(ester-amide) (referred to as an EPTEA resin). The PAOPA resin can be prepared by reacting a monocarboxylic acid, a diamine compound, and a dibasic acid. The EPTEA resin can be prepared by reacting a dibasic acid, a diamine, a polyol and a mono alcohol. Preferably the EPTEA resin may be formed from reaction of : (a) at least 50 equivalent percent of the dibasic acid comprising polymerized fatty acid; (b) at least 50 equivalent percent of the diamine comprising ethylene diamine; (c) 10-60 equivalent percent of the total of the hydroxyl and amine equivalents provided by diamine, polyol and monoalcohol are provided by monoakohol; and (d) no more than 50 equivalent percent of the total of the hydroxyl and amine equivalents provided by diamine, polyol and monoalcohol are provided by polyol. Preparation and description of these resins is found in US 7 329 71982 and US 6 492 458. Particularly preferred are resins under the commercial trademark Sylvaclear PA 1200V, identified by INCI name of Potyamide-3, and Sylvaclear AF 1900V, both sold by Arizona Chemical Company, Jacksonville, Florida.

Not all condensation polymerized polyamides will be suitable resins for purposes of this invention. We have found that activity is only shown by polyamides having an amine number no lower than 0.3, more preferably between 0.3 and 6.0, still more preferably between 0.4 and 4.0, and optimally between 1.0 and 3.0. The amine number is determined by titration with perchloric acid in glacial acetic acid. The end point is determined by a potentiometric method using a glass/electrode pH meter. The method is described by Myers RT "Impurities In High Molecular Weight Amine Products", the Ohio Journal of Science 58(1) January (1958) page 34.

Another physical parameter necessary for effective polyamides of the present invention is an HLB value of at least 16, preferably from 16.2 to 20, more preferably from 17 to 20. The term "HLB" stands for the Hydrophile/Lipophile/Balance. This factor measures the degree to which a surfactant is hydrophilic or lipophilic. Determination of HLB is done by calculating values for different regions of a surfactant molecule. References to calculating by the method may be found in Griffin "Classification of Surface-Active Agents by 'HLB' ", Journal of the Society of Cosmetic Chemists 1 (1949) page 311; Griffin WC "Calculation of HLB Values of Non-Ionic Surfactants", Journal of the Society of Cosmetic Chemists 5 (1954) page 259; Davies JT "A Quantitative Kinitic Theory of Emulsion Type, I. Physical Chemistry of the Emulsifying Agent", Gas/Liquid and Liquid/Liquid Interface, Proceedings of the International Congress of Surface Activity (1957) pages 426-438.

Amounts of the particles within the cosmetic composition may range from 0.1 to 20%, preferably from 2 to 15%, optimally from 4 to 10% by weight of the cosmetic composition.

Average particle size of the particles may range from 10 to 2,000 nm, preferably from 100 to 1,500 nm, and optimally from 200 to 1,000 nm.

Normally polyamide particles of the present invention will be found dispersed within a water phase of a water and oil cosmetic emulsion. These particles generally will not be within the oil phase of the emulsions. Since niacinamide is found in water phases of emulsions, placement of the polyamide particles in the same (water) phase will allow for interaction with niacinamide to achieve benefits of this invention.

Sunscreen agents are added to compositions of the present invention. The sunscreen agent is either solubilized or uniformly dispersed within the condensation polymerized polyamide to form composite particles. Sunscreen agents can also be formed as a core surrounding the polyamide particles.

Relative weight ratio of organic sunscreen agent to polyamide may range from 5:1 to 1:10, preferably from 3:1 to 1:8, more preferably from 2:1 to 1:7, optimally from 1:1 to 1:3. Amounts of the polyamide may range from 10% to 99.5% by weight of the composite particles. More preferably weight of the polyamide may range from 30% to 98%, optimally from 50 to 85% by weight of the composite particles. Amounts of the sunscreen agent may range from 0.5 to 90%, preferably from 2 to 70%, optimally from 30 to 50% by weight of the composite particles.

Sunscreen agents according to this invention will have at least one chromphoric group absorbing within the ultraviolet ranging from 290 to 400 nm. Chromophoric organic sunscreen agents may be divided into the following categories (with specific examples) including: p-aminobenzoic acid, its salts and its derivatives (ethyl, isobutyl, glyceryl esters; p-dimethylaminobenzoic acid); anthranilates (o-aminobenzoates; methyl, menthyl, phenyl, benzyl, phenylethyl, linalyl, terpinyl and cyclohexenyl esters); salicylates (octyl, amyl, phenyl, benzyl, menthyl, glyceryl and dipropyleneglycol esters); cinnamic acid derivatives (menthyl and benzyl esters, alpha-phenyl cinnamonitrile; butyl cinnamoyl pyruvate); dihydroxycinnamic acid derivatives (umbelliferone, methylumbelliferone and methylaceto-umbelliferone); trihydroxycinnamic acid derivatives (esculetin, methylesculetin, daphnetin, and the glucosides, esculin and daphnin); hydrocarbons (diphenylbutadiene and stilbene); dibenzalacetone and benzalacetophenone; naphtholsulfonates (sodium salts of 2-naphthol-3,6-disulfonic and of 2-naphthol-6,8-disulfonic acids); dihydroxy-naphthoic acid and its salts; o- and p-Hydroxybiphenyldisulfonates; coumarin derivatives (7-hydroxy, 7-methyl and 3-phenyl); diazoles (2-acetyl-3-bromoindazole, phenyl benzoxazole, methyl naphthoxazole and various aryl benzothiazoles); quinine salts (bisulfate, sulfate, chloride, oleate and tannate); quinoline derivatives (8-hydroxyquinoline salts and 2-phenylquinoline); hydroxy- or methoxy-substituted benzophenones; uric and vilouric acids; tannic acid and its derivatives (e.g. hexaethylether); (butyl carbityl) (6-propyl piperonyl) ether; hydroquinone; benzophenones (oxybenzone, sulisobenzone, dioxybenzone, benzoresorcinol, 2,2',4,4'-tetrahydroxybenzophenone, 2,2'-dihydroxy-4,4'-dimethoxybenzophenone and octabenzone); 4-isopropyldibenzoylmethane; butylmethoxydibenzoylmethane; etocrylene; and 4-isopropyl-dibenzoylmethane.

Particularly useful sunscreen agents are: 2-ethylhexyl p-methoxycinnamate, 4,4'-t-butyl methoxydibenzoylmethane, 2-hydroxy-4-methoxybenzophenone, octyldimethyl p-aminobenzoic acid, digalloyltrioleate, 2,2-dihydroxy-4-methoxybenzophenone, ethyl 4-[bis(hydroxypropyl)]aminobenzoate, 2-ethylhexyl-2-cyano-3,3-diphenlacrylate, 2-ethylhexylsalicylate, glyceryl p-aminobenzoate, 3,3,5-trimethylcyclohexylsalicylate, methylanthranilate, p-dimethylaminobenzoic acid or aminobenzoate, 2-ethylhexyl p-dimethylaminobenzoate, 2-phenylbenzimidazole-5-sulfonic acid, 2-(p-dimethylaminophenyl)-5-sulfoniobenzoxazoic acid, 4-methylbenzylidene camphor, bis-ethylhexyloxyphenol methoxyphenol triazine, methylene bis-benzotriazolyl tetramethylbutylphenol, dimethicodiethylbenzal malonate, isoamyl methoxycinnamate, octyl triazone, terephthalidene dicamphor sulfonic acid and mixtures thereof.

Cosmetic compositions of this invention may not only have sunscreen agent held within but an amount of sunscreen agent may be formulated free of polyamide within the composition. When present outside the composite, the sunscreen agent may be available in amounts from 0.1 to 25%, particularly from 2 to 15% by weight of the composition. Some preferred embodiments of this invention may be formulated without any sunscreen agent external to the composites or with only a relatively small amount external to the composite particles. For instance the external sunscreen agent may range in amount from 0 to 5%, preferably from 0.01 to 2%, and possibly from 0.01 to 0.8% by weight of the composition.

The composition of the present invention may contain a variety of other components to enhance physical properties and performance.

Compositions of the present invention may include a cosmetically acceptable carrier. The carrier may be a liquid or solid material. Carriers may be present in amounts ranging from 5 to 98%, preferably from 20 to 95%, optimally from 40 to 80% by weight of the cosmetic compositions. Water is the most common carrier for this invention. Oily carriers in the presence of water and an emulsifier will form emulsion systems as carriers. These systems may either be water-in-oil or oil-in-water emulsions. Besides water, suitable carrier classes include silicones, polyhydric alcohols, fatty alcohols, hydrocarbons, triglycerides and thickening powders.

Silicones when present may range from 5% to 60%, more preferably from 5% to 40%, by weight of the composition. These silicones may be organic, silicone-containing or fluorine-containing, volatile or non-volatile, polar or non-polar.

Particularly preferred volatile silicone oils are cyclic volatile silicones wherein the repeating unit ranges from 3 to 5; and linear silicones wherein the repeating unit ranges from 1 to 7. Highly preferred examples of volatile silicone oils include cyclomethicones of varying viscosities, e.g. Dow Corning 200, Dow Corning 244, Dow Corning 245, Dow Corning 344 and Dow Corning 345 (commercially available from Dow Corning Corp.); SF-1204 and SF-1202 Silicone Fluids, GE 7207 and 7158 (commercially available from G.E. Silicones) and SWS-03314 (commercially available from SWS Silicones Corp).

Hydrocarbons may be useful as cosmetically acceptable carriers for compositions of this invention. They may include mineral oil, petrolatum and polyalpha-olefins. Examples of preferred volatile hydrocarbons include polydecanes such as isododecane and isodecane (e.g. Permethyl-99A which is available from Presperse Inc.) and the C₇-C₈ through C₁₂-C₁₅ isoparaffins (such as the Isopar Series available from Exxon Chemicals).

Polyhydric alcohols may serve as carriers. Illustrative of this group are propylyene glycol, dipropylene glycol, polypropylene glycol, polyethylene glycol, sorbitol, hydroxypropyl sorbitol, hexylene glycol, 1,3-butylene glycol, isoprene glycol, ethoxylated glycerol, propoxylated glycerol and mixtures thereof. Most referred is glycerol known also as glycerin.

Fatty alcohols may also be useful carriers. The term "fatty" refers to carbon chain lengths ranging from 10 to 30 carbon atoms. Illustrative of this category are lauryl alcohol, cetyl alcohol, stearyl alcohol, isostearyl alcohol and combinations thereof.

Triglycerides are another group of materials useful as carriers. Illustrative but not limiting are sunflower seed oil, cotton oil, canola oil, soybean oil, castor oil, borage oil, olive oil, shea butter, jojoba oil and mixtures thereof. Mono- and di- glycerides may also be useful. Illustrative of these categories are glyceryl monostearate and glyceryl distearate.

The optional components, when incorporated into the composition, should be suitable for use in contact with human keratinous tissue without undue toxicity, incompatibility, instability, allergic response, and the like within the scope of sound judgment. The CTFA Cosmetic Ingredient Handbook, Second Edition (1992) describes a wide variety of nonlimiting cosmetic and pharmaceutical ingredients commonly used in the skin care industry, which are suitable for use in the compositions of the present invention. Examples of these classes include: abrasives, absorbents, aesthetic components such as fragrances, pigments, essential oils, skin sensates, astringents, etc. (e.g. clove oil, menthol, camphor, eucalyptus oil, eugenol, menthyl lactate and witch hazel distillate), anti-acne agents, anti-caking agents, antifoaming agents, antimicrobial agents, antioxidants, biological additives, buffering agents, bulking agents, chelating agents, chemical additives, colorants, cosmetic astringents, cosmetic biocides, denaturants, drug astringents, external analgesics, film forming polymers, opacifying agents, pH adjusters, propellants, reducing agents, sequestrants, skin bleaching and lightening agents, skin conditioning agents, skin soothing and/or healing agents and derivatives, skin treating agents, and vitamins and derivatives thereof.

In any embodiment of the present invention, the actives useful herein can be categorized by the benefit they provide or by their postulated mode of action. However it is to be understood that the actives useful herein can in some instances provide more than one benefit or operate via more than one mode of action. Therefore classifications herein are made for the sake of convenience and are not intended to limit the active to that particular application or applications listed.

The compositions may comprise a skin lightening agent. When used, the compositions preferably comprise from 0.5% to 10%, more preferably from 1% to 8%, and optimally from 3% to 7%, by weight of the composition of a skin lightening agent. Suitable skin lightening agents in addition to the already present niacinamide may further include kojic acid, arbutin, tranexamic acid, placental extract, ascorbic acid and derivatives thereof (e.g. magnesium ascorbyl phosphate, sodium ascorbyl phosphate, ascorbyl glucoside and ascorbyl tetraisopalmitates). Other skin lightening materials suitable for use herein include Actiwhite® (Cognis), Emblica® (Rona), Azeloglicina (Sinerga) and extracts (e.g. mulberry extract). Most preferred is niacinamide, also known as vitamin 83.

A safe and effective amount of an anti-oxidant/radical scavenger may be added in amounts from 0.01% to 10%, more preferably from 0.1% to 5% by weight of the composition.

Anti-oxidants/radical scavengers may be employed such as ascorbic acid (vitamin C) and its salts, ascorbyl esters of fatty acids, ascorbic acid derivatives (e.g. magnesium ascorbyl phosphate), tocopherol (vitamin E), tocopherol sorbate, tocopherol acetate, other esters of tocopherol, butylated hydroxy benzoic acids and their salts, 6-hydroxy-2,5,7,8-tetramethylchroman-2-carboxylic acid (commercially available under the tradename Rotor®), amines (e.g. N,N-diethylhydroxylamine, amino-guanidine), nordihydroguaiaretic acid, bioflavonoids, amino acids, silymarin, tea extracts and grape skin/seed extracts. Preferred anti-oxidants/radical scavengers are selected from esters of tocopherol, more preferably tocopherol acetate.

The compositions of the present invention may optionally comprise a flavonoid compound. Flavonoids are disclosed in US 5 686 082 and US 5 686 367. Examples of flavonoids particularly suitable flavones, isoflavones, coumarins, chromones, discoumarols, chromanones, chromanols, isomers (e.g. cis/trans isomers) thereof and mixtures thereof.

Preferred for use are flavones and isoflavones, in particular daidzein (7,4'-dihydroxy isoflavone), genistein (5,7,4'-trihydroxy isoflavone), equol (7,4'-dihydroxy isoflavan), 5,7-dihydroxy-4'-methoxy isoflavone, soy isoflavones (a mixture extracted from soy), and mixtures thereof. Flavonoid compounds useful herein are commercially available from a number of sources, e.g. Indofine Chemical Company Inc. Stearloids, Inc. and Aldrich Chemical Company Inc. The herein described flavonoid compounds are preferably present in from 0.01% to 20%, more preferably from 0.1% to 10%, and even more preferably from 0.5% to 5% by weight.

Anti-inflammatory agents useful herein include allantoin and compounds of the licorice (the plant genus/species Glycyrrhiza glabra) family, including glycyrrhetic acid, glycyrrhizic acid and derivatives thereof (e.g. salts and esters).

The compositions may comprise a tanning active. When present, it is preferable that the compositions comprise from 0.1% to 20%, more preferably from 2% to 7% by weight of the composition. A preferred tanning active is dihydroxyacetone.

The compositions may comprise an antimicrobial or antifungal active. Such actives are capable of destroying microbes, preventing the development of microbes or preventing the pathogenic action of microbes. A safe and effective amount of an antimicrobial or antifungal active may be added to the present compositions, preferably from 0.001% to 10%, more preferably from 0.01% to 5%, and even more preferably from 0.05% to 2% by weight of the composition.

Preferred examples of actives include those selected from the group consisting of salicylic acid, benzoyl peroxide, 3-hydroxy benzoic acid, glycolic acid, lactic acid, 4-hydroxy benzoic acid, acetyl salicylic acid, 2-hydroxybutanoic acid, 2-hydroxypentanoic acid, 2-hydroxyhexanoic acid, cis-retinoic acid, trans-retinoic acid, retinol, phytic acid, N-acetyl-L-cystein, lipoic acid, azelaic acid, arachidonic acid, benzoylperoxide, tetracycline, ibuprofen, naproxen, hydrocortisone, acetominophen, resorcinol, -phenoxyethanol, phenoxypropanol, phenoxyisopropanol, 2,4,4'-trichloro-2'-hydroxydiphenyl ether, 3,4,4'-trichlorocarbanilide, octopirox, ciclopirox, lidocaine hydrochloride, dotrimazole, climbazole, miconazole, ketoconazole, neocycin sulfate and mixtures thereof.

The compositions may comprise a conditioning agent selected from the group consisting of humectants, moisturizers or skin conditioners. A variety of these materials can be employed and each can be present at a level of from 0.01% to 40%, more preferably from 0.1% to 30%, and even more preferably from 0.5% to 15% by weight of the composition. These materials include, but are not limited to, guanidine; urea; glycolic acid and glycolate salts (e.g. ammonium and quaternary alkyl ammonium); lactic acid and lactate salts (e.g. ammonium and quaternary alkyl ammonium); aloe vera in any of its variety of forms (e.g., aloe vera gel); polyhydroxy compounds such as sorbitol, mannitol, glycerol, hexanetriol, butanetriol, propylene glycol, butylene glycol and hexylene glycol; polyethylene glycols; sugars and starch derivatives (e.g. alkoxylated glucose, fructose, sucrose, trehalose); hyaluronic acid; lactamide monoethanolamine; acetamide monoethanolamine; sucrose polyester; petrolatum; and mixtures thereof.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

The following examples will more fully illustrate the embodiments of this invention. All parts, percentages and proportions referred to herein and in the appended claims are by weight unless otherwise illustrated.

### EXAMPLES 1-15

A series of comparison experiments were conducted to evaluate skinfeel properties in a number of vanishing cream compositions. For many of the compositions, the formula contained a sunscreen agent; therefore, sunscreen efficacy (SPF) was also evaluated. table I outlines formulas for these compositions.

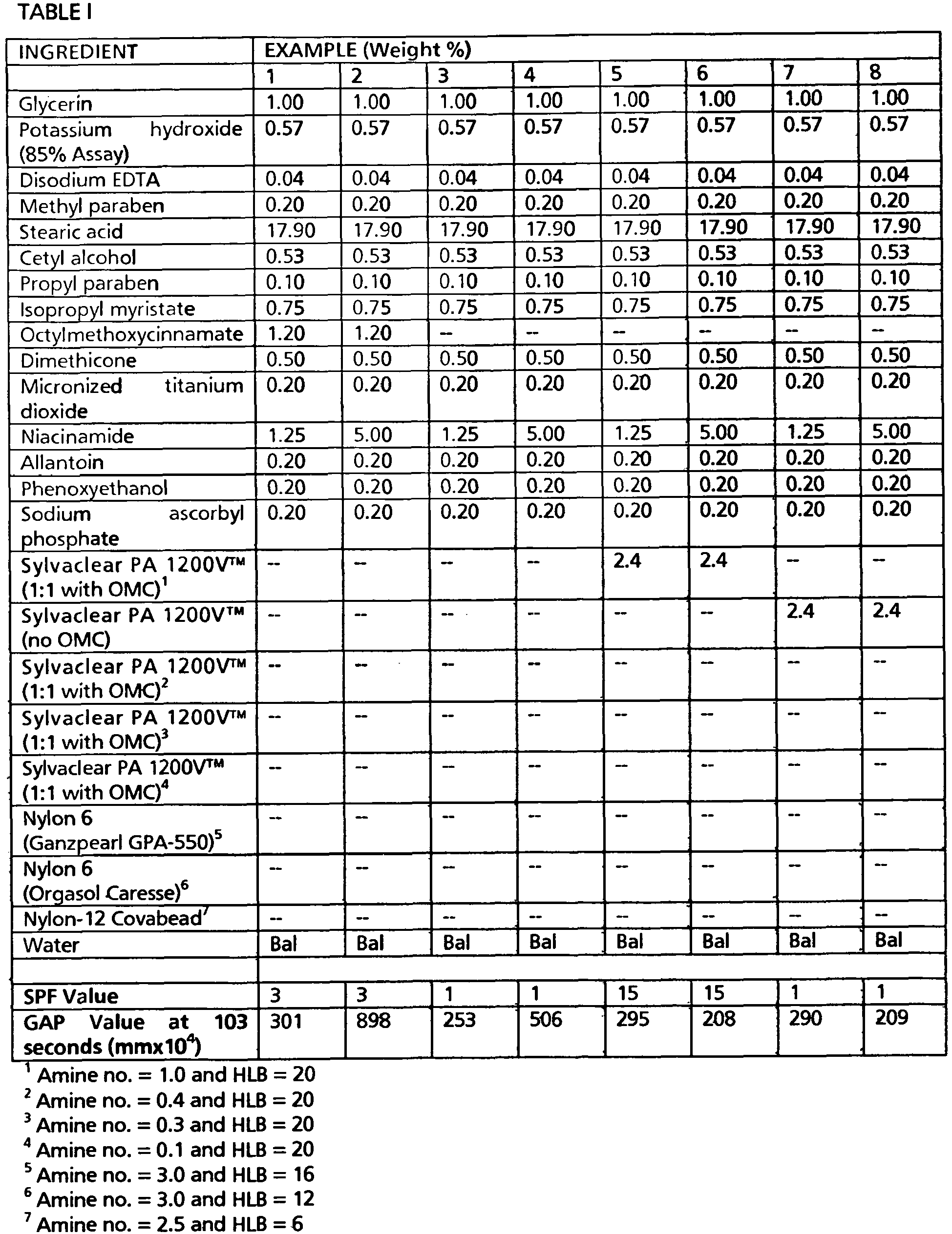

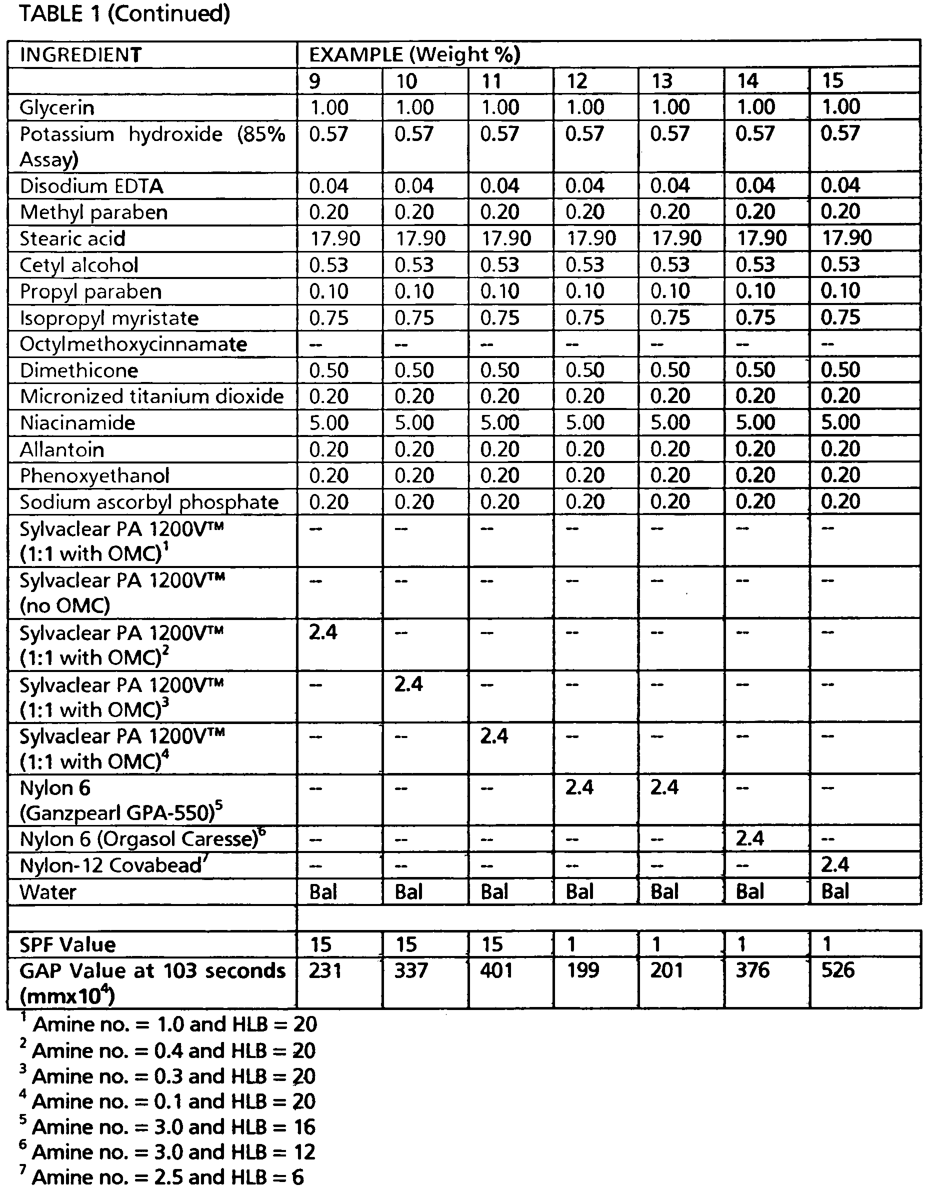

### Test For Skinfeel Properties

A "squeeze flow under-constant force" procedure was used herein to study how easily a lotion/cream is squeezed during application onto the skin. The larger the-Gap Value (sample thickness), the more difficult it is to squeeze the sample. The Gap Value correlates with skinfeel properties as experienced by a consumer in applying the lotion/cream sample to skin. Gap Values must lie between tolerance limits of 175 to 350. Within the tolerance range, the lower the gap value the better the skinfeel.

The procedure is conducted utilizing a Paar Physica MCR 300 rheometer. Samples are compressed between concentric parallel plates (diameter of 25 mm for upper plate) and initial gap (vertical distance between the two plates) of 1 mm. The measurements are performed by squeezing the samples with a constant force of 12 Newton at ambient condition (23-24°C). The time-dependence of sample thickness (gap) is measured over time and, in particular, the Gap Value recorded at 103 seconds (mmx10⁴).

### SYLVACLEAR COMPOSITE PARTICLES

The composite particles that contained the sunscreen agent octylmethoxycinnamate (OMC) were formulated to achieve a sunscreen level of 1.2% by weight in the overall composition. These particles were made in the following manner for use in the examples. A typical process to manufacture composite particles of the present invention is herein described. Water in the amount of 750 gm is charged to a vessel fitted with an Escolabor™ mixer (1 liter model manufactured in Riehn, Switzerland) which has scrape surface blades (scraper). The water is then heated to 60°C. A metal beaker is charged with 125 gm Sylvaclear PA1200V™ and 125 gm Parsol MCX® (2-ethylhexyl-p-methoxy cinnamate referred to as "OMC"). The beaker is placed in a water bath. The resin mixture is heated up to 85-90°C and mixed until homogenous. The resin from the beaker is then added slowly to the vessel of heated water with slow mixing (20% power use on the scraper) followed by a cooling period. Upon reaching 36°C, a preservative mixture is added which includes 2.5 gm Glydant Plus®, 4 gm phenoxyethanol, 2 gm methyl paraben and 2 gm propyl paraben. The combination is then mixed and cooled. Composite particles of resin/sunscreen/preservative are then separated (via centrifuge) as particulates from the water phase.

### RESULTS

Examples 1 and 2 differ only in the amount of niacinamide present. As the level of niacinamide goes from 1.25% up to 5%, there is a steep rise in the GAP Value from 301 to 898. This indicates that niacinamide and stearic acid are adversely interacting to form a relatively hard aesthetically displeasing composition. The same principle is demonstrated for examples 3 and 4. The GAP Value increases significantly as niacinamide content moves from 1.25% up to 5%. These samples unlike examples 1 and 2 do not contain any sunscreen agent.

Examples 5 and 6 are formulated similar to Examples 1 and 2 except for incorporation of 2.4% composite polyamide particles (Sylvaclear PA 1200V™). The GAP Values of 295 and 208, respectively reveal improvement of the underlying rheology to provide a more aesthetic skinfeel.

Examples 7 and 8 demonstrate a similar effect in use of the polyamide particles for lowering GAP Values. Examples 7 and 8 are totally devoid of any sunscreen agent either internal or external to the particles.

Examples 9 and 10 evaluate the effect of amine number. The polyamide Sylvaclear™ comprising the composite particulate has respective amine numbers of 0.4 and 0.3. GAP Value rises from 231 to 337 as the amine number changes from 0.4 to 0.3.

Example 11 illustrates the effect of a polyamide having amine number of 0.1. Again, the GAP Value (401) has further increased to an even more aesthetically non-desirably skinfeel.

Example 12 reveals the effect of utilizing a polyamide with an amine number of 3.0. The GAP Value is a relatively low 199. The sequence of formulas with different amine numbers demonstrate that best results are obtained with polyamides having amine values of at least 0.3 and higher.

Examples 13 and 14 utilize polyamides having identical amine numbers of 3.0. However, the respective HLB values are 16 and 12. It is seen that example 14 with polyamide of HLB being 6 provides a GAP Value significantly higher than the one with HLB of 16. Poor skinfeel performance is confirmed by example 15. Therein the polyamide had an amine number of 2.5 and a HLB of 6. The CAP Value was an unacceptable large 526.

Based on the foregoing results, it appears that polyamide particulates can have an important effect upon regulating the rheological properties of niacinamide compositions thickened with carboxylic acid/salt structurants. Further, the polyamide needs to have an amine number of at least 0.3 and an HLB value of at least 16.

## Claims

1. A cosmetic composition comprising:
(i) from 0.5 to 10% by weight of niacinamide;
(ii) from 0.1 to 30% by weight of a structurant selected from the group consisting of C₁₂-C₂₀ fatty acid or salt thereof, a polymer with repeating carboxylic acid or salt thereof monomer units, and mixtures thereof; and
(iii) from 0.1 to 20% by weight of particles comprising a condensation polymerized polyamide, the polyamide having an amine value of no lower than 0.3 and an HLB value of 16 or higher.
wherein a sunscreen agent is either solubilized or uniformly dispersed within the condensation polymerized polyamide to form composite particles.

2. A composition according to claim 1 wherein the particles have an average particle size ranging from 10 to 2,000 nm.

3. A composition according to claim 2 wherein the particles have an average particle size ranging from 100 to 1,500 nm.

4. A composition according to any one of the preceding claims wherein the polyamide is a polyalkyleneoxypolyamide.

5. A composition according to any one of claims 1 to 3 wherein the polyamide is an ester-terminated poly(ester-amide).

6. A composition according to any one of the preceding claims wherein niacinamide is present in an amount from 1 to 8% by weight of the composition.

7. A composition according to claim 6 wherein niacinamide is present in an amount from 3 to 7% by weight of the composition.

8. A composition according to any one of the preceding claims wherein the amine value ranges from 0.3 to 6.

## Patentansprüche

1. Kosmetische Zusammensetzung, umfassend:
(i) 0,5 bis 10 Gew.-% Niacinamid;
(ii) 0,1 bis 30 Gew.-% eines Strukturierungsmittels, das aus der Gruppe, bestehend aus C₁₂-C₂₀-Fettsäure oder Salz davon, einem Polymer mit sich wiederholenden Monomereinheiten von Carbonsäure oder Salz davon und Gemischen davon, ausgewählt ist, und
(iii) 0,1 bis 20 Gew.-% Partikel, die ein kondensationspolymerisiertes Polyamid umfassen, wobei das Polyamid einen Aminwert von nicht niedriger als 0,3 und einen HLB-Wert von 16 oder höher hat;
wobei ein Sonnenschutzmittel in dem kondensationspolymerisierten Polyamid unter Bildung von Verbundpartikeln solubilisiert oder gleichmäßig dispergiert ist.

2. Zusammensetzung gemäß Anspruch 1, wobei die Partikel eine durchschnittliche Partikelgröße im Bereich von 10 bis 2000 mm haben.

3. Zusammensetzung gemäß Anspruch 2, wobei die Partikel eine durchschnittliche Partikelgröße im Bereich von 100 bis 1500 mm haben.

4. Zusammensetzung gemäß einem der vorangehenden Ansprüche, wobei das Polyamid ein Polyalkylenoxypolyamid ist.

5. Zusammensetzung gemäß einem der Ansprüche 1 bis 3, wobei das Polyamid ein esterterminiertes Poly(esteramid) ist.

6. Zusammensetzung gemäß einem der vorangehenden Ansprüche, wobei Niacinamid in einer Menge von 1 bis 8 Gew.-% der Zusammensetzung vorliegt.

7. Zusammensetzung gemäß Anspruch 6, wobei Niacinamid in einer Menge von 3 bis 7 Gew.-% der Zusammensetzung vorliegt.

8. Zusammensetzung gemäß einem der vorangehenden Ansprüche, wobei der Aminwert im Bereich von 0,3 bis 6 liegt.

## Revendications

1. Composition cosmétique comprenant :
(i) de 0,5 à 10 % en poids de niacinamide ;
(ii) de 0,1 à 30 % en poids d'un structurant choisi dans le groupe constitué par un acide gras en C₁₂-C₂₀ ou un sel de celui-ci, un polymère comprenant des motifs monomères répétitifs d'acide carboxylique ou d'un sel ce celui-ci, et des mélanges de ceux-ci ; et
(iii) de 0,1 à 20 % en poids de particules comprenant un polyamide polymérisé par condensation, le polyamide ayant un indice d'amine non inférieur à 0,3 et un indice HLB supérieur ou égal à 16,
dans laquelle un agent de protection solaire est solubilisé ou uniformément dispersé dans le polyamide polymérisé par condensation pour former des particules de composite.

2. Composition selon la revendication 1, dans laquelle les particules ont une taille de particule moyenne comprise dans la plage allant de 10 à 2000 nm.

3. Composition selon la revendication 2, dans laquelle les particules ont une taille de particule moyenne comprise dans la plage allant de 100 à 1500 nm.

4. Composition selon l'une quelconque des revendications précédentes, dans laquelle le polyamide est un polyalkylèneoxypolyamide.

5. Composition selon l'une quelconque des revendications 1 à 3, dans laquelle le polyamide est un poly(ester-amide) à terminaison ester.

6. Composition selon l'une quelconque des revendications précédentes, dans laquelle le niacinamide est présent en une quantité de 1 à 8 % en poids de la composition.

7. Composition selon la revendication 6, dans laquelle le niacinamide est présent en une quantité de 3 à 7 % en poids de la composition.

8. Composition selon l'une quelconque des revendications précédentes, dans laquelle l'indice d'amine est compris dans la plage allant de 0,3 à 6.
